# EUROPÄISCHE PATENTSCHRIFT

(11) **EP 2 601 909 B1**
(45) Veröffentlichungstag und Bekanntmachung des Hinweises auf die Patenterteilung: **14.05.2014**
(21) Anmeldenummer: 12175638.1
(22) Anmeldetag: 10.07.2012
(51) Int. Cl.: A61F 2/18

(54) **Längenvariable passive Gehörknöchelchenprothese**
Passive ossicular prosthetics with variable length
Prothèse d'osselets passive variable en longueur

(30) Priorität: 07.12.2011 DE 202011052221 U
(43) Veröffentlichungstag der Anmeldung: 12.06.2013
(73) Patentinhaber: Heinz Kurz GmbH Medizintechnik, 72144 Dusslingen (DE)
(72) Erfinder: Steinhardt, Uwe, 72145 Hirrlingen (DE); Prof. Dr. Dr. Lenarz, Thomas, 30559 Hannover (DE); Lang, Axel, 71229 Leonberg (DE); Kurz, Heinz, 72144 Dusslingen (DE)
(74) Vertreter: Kohler Schmid Möbus

(56) Entgegenhaltungen:
- DE-B3-102005 010 705
- DE-B3-102010 046 457
- DE-B4-102007 041 539
- DE-C1- 10 047 388

## Beschreibung

Die Erfindung betrifft eine passive Gehörknöchelchenprothese, die mindestens ein Glied der menschlichen Gehörknöchelchenkette ersetzt oder überbrückt, wobei die Gehörknöchelchenprothese an einem Ende ein erstes Befestigungselement zur mechanischen Verbindung mit dem mit dem Trommelfell oder einem Glied der Gehörknöchelchenkette, insbesondere mit dem Ambossfortsatz oder mit dem Hammergriff, und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes, längliches Verbindungselement umfasst, welches ein als Aufnahmeteil längs der Schaftachse des länglichen Verbindungselements ausgebildetes erstes Teilstück und ein als in eine Aufnahmeöffnung des Aufnahmeteils einschiebbares Einschiebeteil ausgebildetes zweites Teilstück aufweist, wobei das erste Befestigungselement mit einem Ende und das zweite Befestigungselement mit dem axial entgegen gesetzten anderen Ende des Verbindungselements mechanisch fest verbunden ist.

Eine derartige Vorrichtung ist bekannt aus der DE 10 2007 041 539 B4, die dem Oberbegriff von Anspruch 1 Zugrundeliegt.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Feisenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöcheichenkette auf das Innenohr übertragen, was zur Schwerhörigkeit führt.

Gehörknöchelchenprothesen dienen der Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommel-fell und dem Innenohr nur begrenzt ermöglicht, weil sie die natürlichen anatomischen Ausbildungen der Gehörknöchelchenkette nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Gehörknöchelchenprothese entweder eine ausreichend große Auswahl von Prothesen unterschiedlicher axialer Länge bereitgehalten werden oder die verwendeten Gehörknöchelchenprothesen müssen während der Operation ausgehend von einer maximalen Ausgangslänge auf die erforderliche axiale Endlänge gebracht werden können.

In der WO 92/18066 A1 ist eine selbstanpassende Gehörknöchelchenprothese beschrieben, die einen komplizierten und in der Herstellung sehr aufwändigen Federmechanismus in der Verbindung zwischen dem ersten und dem zweiten Befestigungselement aufweist, welcher eine ständige Änderung der axialen Länge der Prothese je nach relativer Lage der Befestigungspunkte im Mittelohr bewirkt. Eine reproduzierbar exakte, feste Längeneinstellung de Prothese, die auch nach dem operativen Einsetzen derselben ins Mittelohr erhalten bleibt, ist damit nicht möglich. Zudem erfordert die bekannte Prothese wegen ihres sehr speziellen mechanischen und geometrischen Aufbaus einen erheblichen Platzbedarf im Mittelohr, so dass sie in vielen Fällen aufgrund der individuellen Gegebenheiten beim Patienten gar nicht einsetzbar ist. Außerdem wird Konstruktionsbedingt nach dem Einsetzen ein nicht unerheblicher permanenter Druck zwischen den beiden Befestigungspunkten im Mittelohr aufgebaut, was einer Heilung nach der Operation nicht gerade förderlich ist und auf Dauer häufig zu postoperativen Komplikationen führt.

Eine Gehörknöchelchenprothese mit während der Operation in bestimmten Grenzen variierbarer axialer Länge ist in der DE 39 01 796 A1 beschrieben. Dabei wird die Längenänderung durch ein Verbiegen des als dünner aus Golddraht gefertigten Verbindungselements erreicht, was allerdings einerseits umständlich in der Handhabung, andererseits ziemlich ungenau ist, so dass damit keine exakte Einstellung der gewünschten axialen Länge der Gehörknöcheichenprothese erreicht werden kann. Außerdem ist das Ergebnis bei dieser Technik nicht immer reproduzierbar und es kann sogar vorkommen, dass sich nach dem Verbiegen des Verbindungselements die eingestellte axiale Länge der Gehörknöchelchenprothese durch ein Zurückfedern des Verbindungselements wieder ändert.

Die EP 0 998 884 B1 beschreibt eine Gehörknöchelchenprothese, bei welcher das als länglicher Schaft ausgebildete erste Verbindungselement durch eine Durchgangsbohrung des als Kopfplatte ausgebildeten ersten Befestigungselements hindurch gesteckt wird, bis eine gewünschte Schaftlänge zwischen dem ersten und dem zweiten Befestigungselement erreicht ist. Sodann wird der Schaft in dieser Position durch Verengen der Durchgangsbohrung in der Kopfplatte fixiert und der über die Kopfplatte hinaus überstehende Teil des Schaftes abgelängt. So erhält man auf einfache Weise eine Prothese mit der jeweils gewünschten bzw. erforderlichen, insbesondere nach der Operation exakt gleich bleibenden axialen Länge. Eine ähnlich wirkende Konstruktion ist beispielsweise auch aus der DE 100 45 158 A1 bekannt.

Aus der DE 10 2005 010 705 B3 ist eine Gehörknöchelchenprothese bekannt, bei der eine intraoperative Variabilität der Prothesenlänge dadurch erreicht wird, dass das längliche Verbindungselement in Form einer Kugelkette ausgebildet ist. Diese wird während der Operation mit einer bestimmten Anzahl von Kugeln durch eine Aufnahmeöffnung des ersten Befestigungselements hindurch gesteckt. Danach wird die Kugelkette in der Aufnahmeöffnung des Befestigungselements durch beiderseits der Kugelkette angreifende federnde Stegelemente fixiert, die allerdings - im Gegensatz zur in der gattungsbildenden, eingangs zitierten DE 10 2007 041 539 B4 beschriebenen Prothese - nicht längs der Schaftachse angeordnet sind, sondern quer dazu in der Ebene des als Trommelfell-Kopfplatte ausgebildeten ersten Befestigungselements. Diese als Aufnahmeteil für die letzte Kugel wirkenden Stegelemente können daher der als Einschiebeteil wirkenden Kugelkette und mithin dem länglichen Verbindungselement auch nicht annähend die Lage-Stabilität in Richtung der Schaftachse bieten, wie etwa die beiden koaxial zur Schaftachse gegeneinander verschiebbaren Teilstücke des Verbindungselements gemäß der DE 10 2007 041 539 B4.

Der durch die Aufnahmeöffnung ragende, überstehende Teil der Kugelkette bei der Gehörknöchelchenprothese nach der DE 10 2005 010 705 B3 wird vor der Implantation ins Mittelohr abgezwickt, so dass die Prothese am Ende schließlich genau die gewünschte axiale Länge aufweist. In ähnlicher Weise wird eine Längenvariabilität auch bei einer Gehörknöchelchenprothesen nach der DE 10 2005 048 618 B4 erreicht, wobei hier wiederum eine abzwickbare Kugelkette als Verbindungselement verwendet wird, jedoch die Aufnahme im ersten Befestigungselement anders gestaltet ist.

Eine weitere Gehörknöchelchenprothese mit intraoperativ veränderbarer axialer Länge ist in der US-A 3,710,399 beschrieben. Hier wird ein zweigeteiltes Verbindungselement zwischen den beiden Befestigungselementen verwendet, das zwei parallel verlaufende gerade Drahtstücke umfasst, von denen das eine vom ersten und das andere vom zweiten Befestigungselement wegragt. Die beiden Drahtstücke können entweder mittels Drahtschlingen an ihren Enden mit dem jeweils andern Drahtstück verbunden oder in eine Art Verbindungsmuffe mit zwei parallelen Längsbohrungen für die beiden Drahtstücke gesteckt werden. Im ersteren Fall ist jedoch die Fixierungsposition und damit die relative Lage der beiden Drahtstücke nur sehr ungenau einzustellen, so dass eine exakte und reproduzierbare Längeneinstellung der Prothese nicht möglich ist. Im zweiten Fall kann es nach dem Einstecken der Drahtstücke in die Verbindungsmuffe leicht zu Verkippungen, Verknickungen oder Verschiebungen der relativen Lagen der Drahtstücke zueinander kommen, wodurch ebenfalls eine genaue Einstellung der axialen Prothesenlänge erschwert bzw. unmöglich gemacht wird.

Wiederum eine andere Technik der Längeneinstellung wird bei einer Gehörknöchelchenprothese angewendet, wie sie aus der DE 10 2005 027 215 A1 bekannt ist. Diese Prothese zielt ausschließlich auf die Situation einer Steigbügel-Operation ab, so dass immer ein kolbenförmiges Piston als zweites Befestigungselement vorgesehen ist. In diesem Piston ist ein Aufnahmemechanismus angeordnet, in welche das schaftförmige Verbindungselement in axialer Richtung eingeschoben werden soll. Von dem Verbindungselement radial abgespreizte Blattfedern sollen dann in einer gewünschten relativen Position zwischen Verbindungselement und zweitem Befestigungselement eine Arretierung bewirken. Abgesehen davon, dass eine exakt reproduzierbare Einstellung einer gewünschten axialen Länge der Prothese damit nicht immer garantiert sein dürfte, ist der Anwendungsbereich dieser Gehörknöchelchenprothese lediglich auf Steigbügel-Operationen beschränkt, bei der über das Piston eine unmittelbare Verbindung zum Innenohr hergestellt wird. Soll jedoch als zweites Befestigungsteil beispielsweise eine Glocke, ein Stempel, ein Clip oder ein flacher Schuh für eine Verbindung mit einem anderen Teil der Gehörknöchelchenkette verwendet werden, ist diese bekannte Prothese nicht verwendbar. Wenn man nämlich einen entsprechenden Aufnahmemechanismus im zweiten Befestigungsteil unterbringen will, so funktioniert dies schon aus geometrischen Gründen nur in einem Kolben, aber niemals in einer Glocke, einem flachen Schuh oder gar in einem Clip.

Die in der US-A 5,554,188 beschriebene Gehörknöchelchenprothese umfasst wiederum ein als zweigeteilter Schaft aufgebautes Verbindungselement, bei dem der erste, stangenförmige Abschnitt in eine Aufnahmebohrung des als Aufnahmeteil ausgebildeten zweiten Abschnitts einführbar und axial in der Bohrung verschiebbar ist. Um ..' eine gewünschte axiale Länge der Prothese zu erhalten, wird der stangenförmige erste Abschnitt ausgehend von einer maximalen Ausgangslänge auf eine geeignete Endlänge abgeschnitten und bis auf Anschlag in den zweiten Abschnitt eingeschoben. Durch eine entsprechende Gestaltung des lichten Durchmessers der Aufnahmebohrung relativ zum Außendurchmesser des ersten Abschnitts soll nun eine friktionale Verklemmung von erstem und zweitem Abschnitt eine gewisse Fixierung der Prothesenlänge bewirken, wobei die eigentliche Fixierung dadurch erreicht wird, dass sich die gegeneinander beweglichen Teile der Prothese nach der operativen Einführung ins Mittelohr aufgrund ihres jeweiligen Anschlages an den beiden Befestigungspunkten nicht allzu weit voneinander entfernen können. Eine exakt gleich bleibende Prothesenlänge kann damit allerdings auf Dauer nicht sichergestellt werden.

Eine einteilige, längenvariable Gehörknöchelchenprothese ist bekannt aus der DE 10 2009 016 468 B3. Hier umfasst die Verstellvorrichtung mindestens zwei symmetrisch zur Längsachse des Verbindungselements verlaufende, in Achsrichtung streckbare und/oder stauchbare Teilstränge, die dauerhaft bleibend plastisch verformbar und zumindest vor ihrem Verformen quer zur Längsachse in mehreren Schlaufen gefaltet sind.

Bei der gattungsbildenden, in der eingangs zitierten DE 10 2007 041 539 B4 beschriebenen Gehörknöchelchenprothese schließlich ist das Verbindungselement in axialer Richtung zwischen dem Aufnahmeteil und dem Einschiebeteil Längenvariabel gestaltet, wobei die Festlegung der konkreten axialen Länge des Verbindungselements einer individuellen Gehörknöchelchenprothese durch Verklemmen des Einschiebeteiles mit dem Aufnahmeteil in einer gewünschten relativen koaxialen Einschiebeposition erfolgt und die Klemmkraft F_{K} zwischen dem Aufnahmeteil und dem Einschiebeteil im verklemmten Zustand mindestens 10mal, vorzugsweise etwa 100mal größer ist als die maximalen, natürlicherweise im Mittelohr im Bereich der Gehörknöchelchen auftretenden äußeren Kräfte. Damit lässt sich eine gewünschte, definierte Länge der Prothese auch schon vor einem Einklemmen zwischen den beiden Befestigungspunkten herstellen, wobei diese Länge auch nach Abschluss der Operation, beispielsweise nach dem Durchstecken eines als Piston ausgebildeten zweiten Befestigungselements durch eine perforierte Steigbügel-Fußplatte, fix beibehalten wird. Dies ermöglicht unaufwändig und kostengünstig eine echte Längenvariabilität der Gehörknöchelchenprothese "in situ" bzw. intraoperativ, wobei weder größere Sortimente von Prothesen unterschiedlicher Längen während jeder Operation bereitgehalten werden müssen. Außerdem ist die Einstellung der jeweils gewünschten individuellen Prothesenlänge und damit deren Handhabung besonders einfach. Dadurch werden die Vorteile der oben beschriebenen bekannten Gehörknöchelchenprothese gemäß der US-A 5,554,188 genutzt, wobei aber auch die Vorteile der in den oben genannten weiteren Druckschriften beschriebenen längenvariablen Prothesen erhalten bleiben und die gemeinsamen Nachteile vermieden werden. Nachträgliche postoperative unerwünschte Längen- und/oder Lageänderungen der Prothese werden durch die erfindungsgemäß vorgeschriebene Wahl der Klemmkraft F_{K} sicher vermieden. Zudem ist diese Gehörknöchelchenprothese universell bei allen denkbaren Arten von Ankoppefungen im Mittelohrraum einsetzbar und nicht auf eine bestimmte Klasse von Operationen beschränkt, während beispielsweise die Prothese gemäß der oben zitierten DE 10 2005 027 215 A1 ausschließlich in der Situation einer Steigbügel-Operation verwendet werden kann.

Aufgabe der vorliegenden Erfindung ist es demgegenüber, eine gattungsgemäße Gehörknöchelchenprothese der eingangs beschriebenen Art mit möglichst einfach zu realisierenden technischen Mitteln dahin gehend zu verbessern, dass die oben beschriebenen Vorteile der aus der DE 10 2007 041 539 B4 bekannten Prothese beibehalten bleiben, wobei darüber hinaus die Herstellung der Prothese mechanisch noch wesentlich einfacher sein soll und sich die gewünschte Einstellung der Endposition besonders genau und reproduzierbar bewerkstelligen lässt.

Erfindungsgemäß wird diese Aufgabe auf ebenso überraschend einfache wie wirkungsvolle Art und Weise dadurch gelöst, dass das Aufnahmeteil geometrisch so gestaltet ist, dass es einen Endabschnitt des Einschiebeteils mit zwei gegenüber liegenden, im Wesentlichen parallelen und im Wesentlichen parallel zur Schaftachse des länglichen Verbindungselements angeordneten Schenkeln klammerartig kraft- und/oder formschlüssig umgreift, dass die beiden Schenkel jeweils eine Rastereinrichtung aufweisen, die im Zusammenwirken der beiden Schenkel ermöglicht, den umgriffenen Endabschnitt auf wählbaren diskreten räumlichen Positionen relativ zur Schaftachse des länglichen Verbindungselements zu fixieren, dass die Rastereinrichtungen der Schenkel des Aufnahmeteils auf den im implantierten Zustand der Gehörknöchelchenprothese am Außenumfang des Endabschnitts anliegenden Innenflächen jeweils an gegenüber liegenden axialen Positionen der beiden Schenkel Einkerbungen oder Einbuchtungen zu einer - vorzugsweise formschlüssigen - Aufnahme des Endabschnitts aufweisen, und dass die Einkerbungen oder Einbuchtungen auf den Innenflächen der beiden Schenkel jeweils in axialer Richtung der Schenkel äquidistant angeordnet sind, was fertigungstechnisch besonders günstig ist.

Die Konstruktion von zwei parallelen, klammerartigen Schenkeln ist für sich gesehen auch schon bekannt aus der DE 10 2010 046 457 B3, jedoch dort ausschließlich in Zusammenhang mit einer Anordnung zum Einstellen und Fixieren der Relativlage zwischen einerseits einem Aktor-Endstück eines aktiven Hör-Implantats und andererseits einem Glied der Gehörknöchelchenkette oder einem Ankoppelteil zum menschlichen Innenohr. Ein solches *aktives* Hör-Implantat ist mit seinem notwendigen Elektronikteil und seiner Positionierung relativ zum menschlichen Mittelohr völlig anders aufgebaut und wirkt in ganz anderer Weise als eine zur vorliegenden Erfindung gattungsgemäße *passive* Gehörknöchelchenprothese, so dass es demgegenüber als gattungsfremd anzusehen ist und einem einschlägigen Fachmann keine Anregungen zur Verbesserung einer passiven Gehörknöchelchenprothese zu geben vermag.

Die in der erfindungsgemäßen Gehörknöchelchenprothese von den beiden Schenkeln gebildete Klammer ist besonders leicht herzustellen und zudem mechanisch deutlich robuster als das hohle Aufnahmeteil, wie es in der aus der DE 10 2007 041 539 B4 bekannten gattungsgemäßen Prothese verwendet wird. Durch die offene Gestaltung der Klammer lassen sich auch leichter die Position und der Zustand des Einschiebeteils innerhalb des Aufnahmeteils optisch erkennen und gegebenenfalls problemlos korrigieren.

Wenn die diskreten axialen Klemmpositionen zwischen den beiden Schenkeln geometrisch fein genug abgestuft sind, lässt sich damit eine äußerst exakte räumliche Einstellung des Ankoppelpunkts zwischen dem Endabschnitt des Einschiebeteils und dem Aufnahmeteil bewirken, welche andererseits auch die Möglichkeit der räumlichen Positionierung und Fixierung der gewünschten Lage des zweiten Befestigungselements relativ zum ersten Befestigungselement im Mittelohr erheblich verbessert.

Um eine erhöhte Flexibilität bzw. Variabilität der Prothese zu erreichen, kann bei bevorzugten Ausführungsformen der Erfindung das

Verbindungselement mindestens ein Gelenk, insbesondere ein Kugelgelenk aufweisen. Vorteilhaft im Hinblick auf eine besonders hohe postoperative Beweglichkeit der Prothese sind Weiterbildungen, bei denen eine Vielzahl von aneinander angrenzenden weiteren Drehelementen vorgesehen ist, vorzugsweise eine Kugelgelenkkette.

Eine vorteilhafte Weiterbildung dieser Ausführungsformen zeichnet sich dadurch aus, dass das Aufnahmeteil an seinem den beiden Schenkeln abgewandten Ende über ein Kugelgelenk verschwenkbar mit dem ersten Befestigungselement verbunden ist.

Alternativ oder ergänzend kann bei anderen Weiterbildungen das Einschiebeteil an seinem dem Endabschnitt abgewandten Ende über ein Kugelgelenk verschwenkbar mit dem zweiten Befestigungselement verbunden sein.

Besonders viele Freiheitsgrade bezüglich der räumlichen Einstellung des Prothesenmittelteils ermöglicht eine Ausführungsform, bei welcher das Verbindungselement mehrere Aufnahmeteile mit jeweils im Wesentlichen parallelen Schenkeln aufweist, welche jeweils einen Endabschnitt eines Einschiebeteils klammerartig umgreifen. Insbesondere in Kombination mit den oben beschriebenen Ausführungsformen mit Kugelgelenken ergibt sich hier eine schier unendliche Anzahl von geometrischen Einstellungen des Prothesenoberteils relativ zum Prothesenunterteil, was eine sehr präzise intraoperative Anpassung der dem Operateur als Standardteil vorliegenden erfindungsgemäßen Gehörknöchelchenprothese an die individuelle Situation und spezifische Ausprägung des Mittelohrbereichs des jeweiligen Patienten möglich macht, ohne dass eine große Palette unterschiedlich geformter Prothesen in diversen Größen während jeder Operation vorgehalten werden müsste.

Um eine besonders gute und dauerhaft stabile Passung der Anordnung nach dem Einstellen der gewünschten relativen Endposition erstem und zweitem Verbindungselement zu gewährleisten, weist bei bevorzugten Ausführungsformen der Endabschnitt des Einschiebeteils eine auf seinem Außenumfang azimutal verlaufende Ringnut auf, die geometrisch so gestaltet sind, dass sie als Gegenstück zu einer der Einkerbungen oder Einbuchtungen der Rastereinrichtungen der beiden Schenkel des klammerartigen des Aufnahmeteils passt.

Das Einschiebeteil kann kontinuierlich oder in wählbaren diskreten relativen Positionen auf einer axialen Strecke längs der beiden Schenkel mit dem Aufnahmeteil verklemmt werden, so dass sich damit jede gewünschte Prothesenlänge unterhalb der vom Grundmuster der Prothese vorgegebenen Maximallänge individuell genau einstellen lässt.

Besonders einfach zu handhaben sind Varianten, bei denen das Einschiebeteil auf der gewählten relativen koaxialen Klemmposition einrastend mit dem Aufnahmeteil verklemmt werden kann, so dass die Prothese in der gewünschten und eingestellten Länge dauerhaft fixiert bleibt und so zwischen den Befestigungspunkten im Mittelohr positioniert werden kann.

Bei einer Klasse von Ausführungsformen der erfindungsgemäßen Gehörknöchelchenprothese ist das Einschiebeteil mit dem Aufnahmeteil passiv verklemmbar mittels einer eigenen Federwirkung im Zusammenwirken der beiden Schenkel des Aufnahmeteils.

Alternativ dazu kann aber auch bei einer weiteren Klasse von Ausführungsformen der Erfindung das Einschiebeteil mit dem Aufnahmeteil mittels einer äußeren Einwirkung auf die beiden Schenkel des Aufnahmeteils aktiv verklemmbar gestaltet sein.

In einfachen Weiterbildungen dieser Ausführungsformen kann das Einschiebeteil durch Krafteinwirkung von außen, insbesondere mittels Einwirkung einer Crimpzarige, mit dem Aufnahmeteil aktiv verklemmbar sein.

Bei eleganteren, allerdings in der Herstellung dafür auch etwas aufwändigeren Weiterbildungen ist vorgesehen, dass das Einschiebeteil durch Wärmeeintrag auf die Gehörknöchelchenprothese von außen, insbesondere mittels Erwärmung der Gehörknöchelchenprothese auf Temperaturen bis zu 90°C, vorzugsweise auf Körpertemperatur, mit dem Aufnahmeteil aktiv verklemmbar ist.

Ganz besonders vorteilhaft sind Varianten dieser Weiterbildungen, bei denen das Einschiebeteil und/oder das Aufnahmeteil ganz oder teilweise aus einem Material mit Formgedächtnis (=memory effect), insbesondere aus Nitinol hergestellt ist. Die Verwendung derartiger Materialien ist auf dem Gebiet der Gehörknöchelchenprothesen an sich bekannt, erweist sich aber gerade im Zusammenhang mit der vorliegenden Erfindung als besonders wirkungsvoll.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein.

In vielen Ausführungsformen der Erfindung wird das erste Befestigungselement eine zur Anlage am Trommelfell ausgebildete Kopfplatte umfassen. Bei anderen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über die Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über ein als Kolben (=piston) ausgeführtes zweites Befestigungselement.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgeführtes erstes Befestigungselement der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können postoperative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solches "mechanisches Tuning" kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:

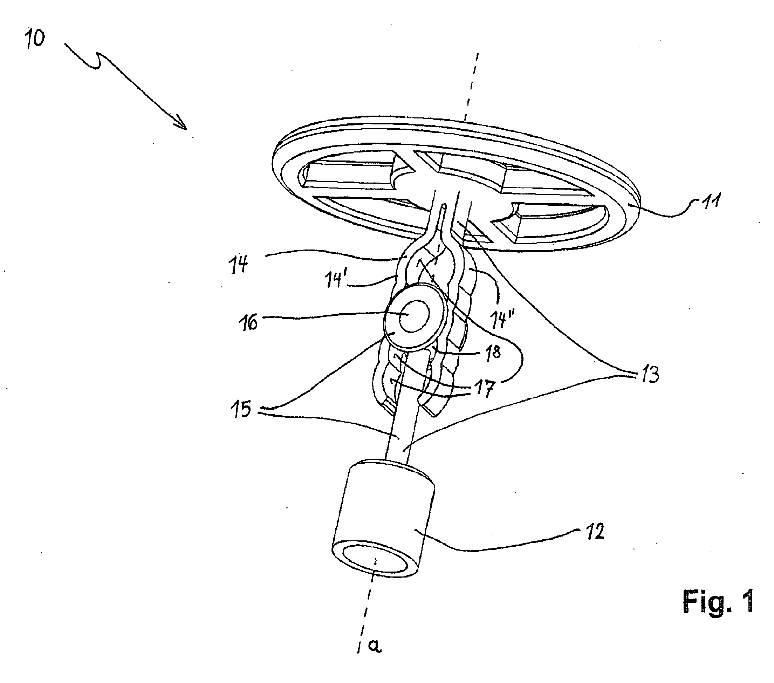

- Fig. 1: eine schematische räumliche Darstellung einer ersten Ausführungsform der erfindungsgemäßen Gehörknöchelchenprothese mit einer Trommelfell-Kopfplatte als erstem Befestigungselement und einem stempelförmigen zweiten Befestigungselement zur Auflage auf der Steigbügel-Fußplatte sowie mit parallel zur Schaftachse des länglichen Verbindungselements angeordneten Schenkeln des klammerartigen Aufnahmeteils;

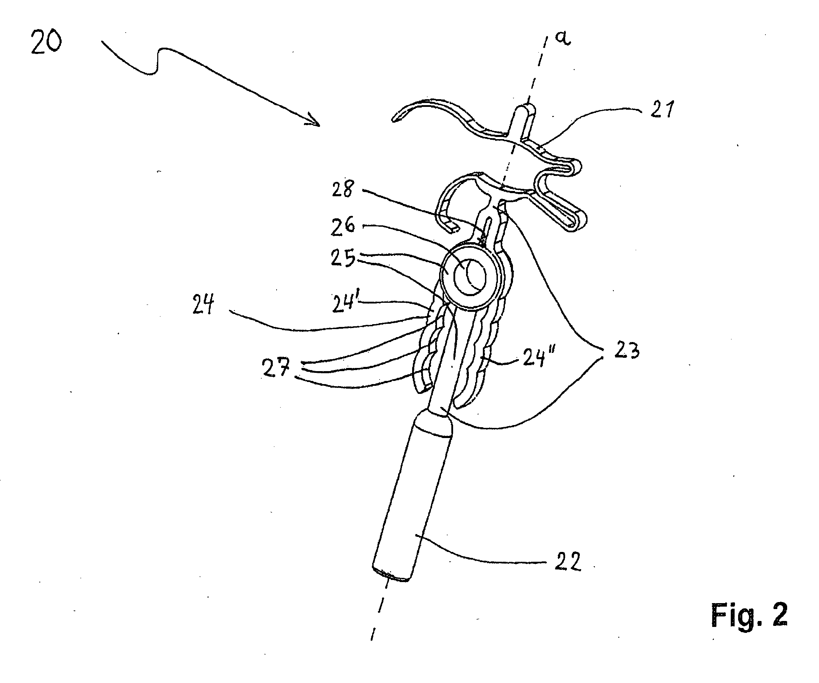

- Fig. 2: eine Ausführungsform mit einem Clip als erstem und einem Piston als zweitem Befestigungselement;

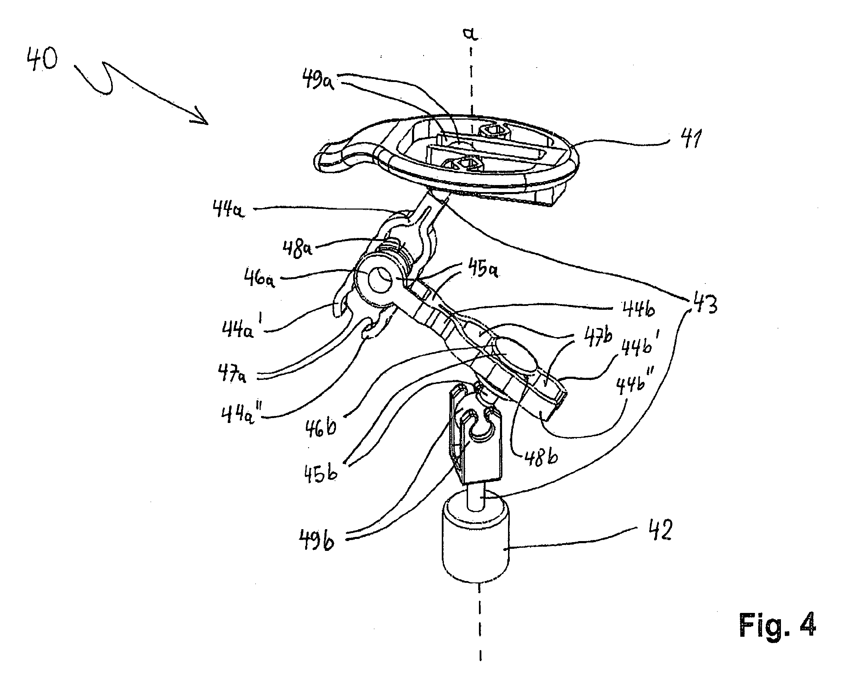

- Fig. 4: eine Ausführungsform mit einer Trommelfell-Kopfplatte als erstem Befestigungselement und einem stempelförmigen zweiten Befestigungselement, sowie mit zwei Aufnahmeteilen mit jeweils parallelen Schenkeln, die jeweils einen Endabschnitt eines Einschiebeteils klammerartig umgreifen, wobei das erste Aufnahmeteil an seinem den beiden Schenkeln abgewandten Ende über ein erstes Kugelgelenk verschwenkbar mit dem ersten Befestigungselement verbunden ist und das zweite Einschiebeteil an seinem dem Endabschnitt abgewandten Ende über ein zweites Kugelgelenk verschwenkbar mit dem zweiten Befestigungselement verbunden ist; und

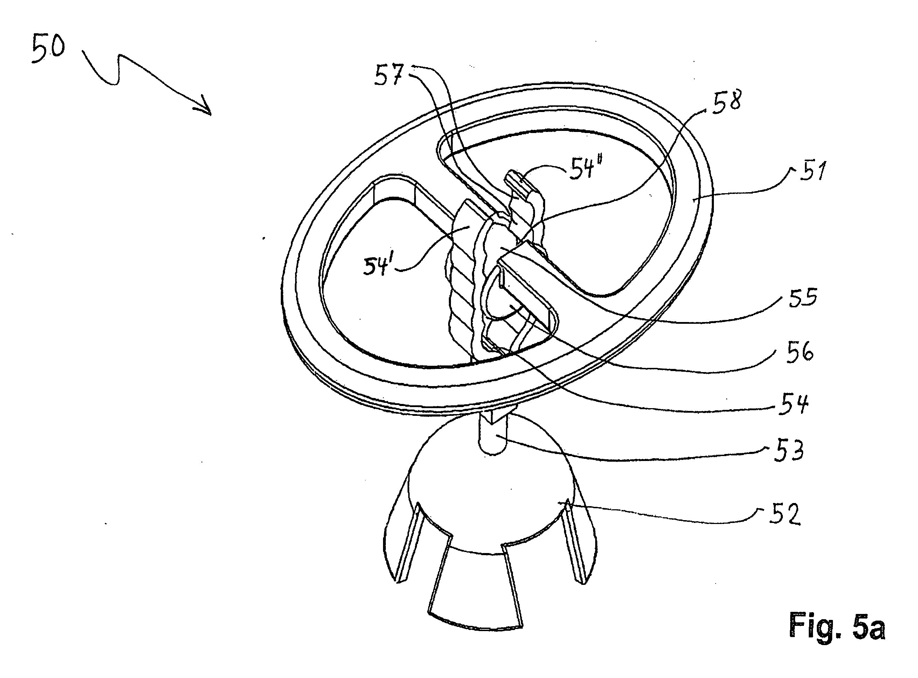

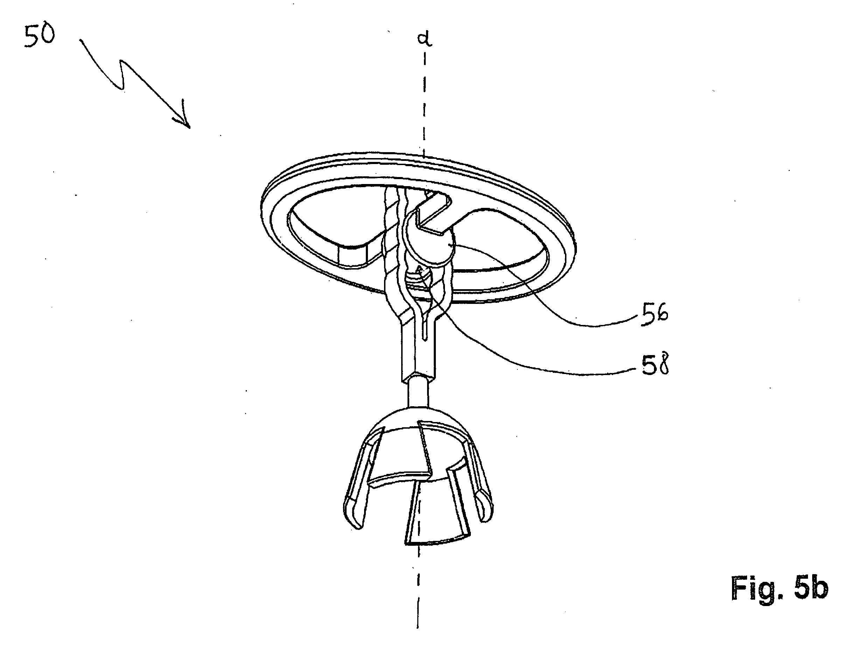

- Fign. 5a,b: eine Ausführungsform mit einer Trommelfell-Kopfplatte als erstem Befestigungselement, einer geschlitzten Glocke als zweitem Befestigungselement sowie einem mit dem zweiten Befestigungselement starr verbundenen Aufnahmeteil.

Die in den Figuren der Zeichnung schematisch dargestellten vier - im Detail unterschiedlich gestalteten - Ausführungsformen der erfindungsgemäßen **Gehörknöchelchenprothese 10; 20; 40; 50** weisen am einen Ende jeweils ein **erstes Befestigungselement 11; 21; 41; 51** auf, welches der mechanischen Verbindung der Prothese mit dem Trommelfell oder einem Glied der Gehörknöchelchenkette dient. Am anderen Ende der Gehörknöchelchenprothese 10; 20; 40; 50 sitzt jeweils ein **zweites Befestigungselement 12; 22; 42; 52** zur mechanischen Verbindung der Prothese mit einem weiteren Glied oder Teilen eines Gliedes der Gehörknöchelchenkette oder zum direkten Eintauchen ins Innenohr. Dazwischen ist ein die beiden Befestigungselemente 11; 21; 41; 51 bzw. 12; 22; 42; 52 Schall leitend miteinander verbindendes **Verbindungselement 13; 23; 43; 53** in Form eines länglichen Schaftes angeordnet. Dieses umfasst jeweils mindestens ein als **Aufnahmeteil 14; 24; 44a, 44b; 54** ausgebildetes erstes Teilstück sowie mindestens ein als in eine Aufnahmeöffnung eines Aufnahmeteils 14; 24; 44a, 44b; 54 einschiebbares, mit dem Aufnahmeteil 14; 24; 44a, 44b; 54 verklemmbares **Einschiebeteil 15; 25; 45a, 45b; 55** ausgebildetes zweites Teilstück, wobei jeweils das erste Befestigungselement 11; 21; 41; 51 mit einem Ende und das zweite Befestigungselement 12; 22; 42; 52 mit dem axial entgegen gesetzten anderen Ende des Verbindungselements 13; 23; 43; 53 mechanisch fest verbunden ist.

Erfindungsgemäß ist das Aufnahmeteil 14; 24; 44a, 44b; 54 geometrisch so gestaltet ist, dass es einen **Endabschnitt 16; 26; 46a, 46b; 56** des Einschiebeteils 15; 25; 45a, 45b; 55 mit zwei gegenüber liegenden, im Wesentlichen parallelen **Schenkeln 14',14"; 24',24"; 44a', 44a"; 44b',44b"; 54',54"** klammerartig kraft- und/oder formschlüssig umgreift, wobei die beiden Schenkel 14',14"; 24',24"; 44a', 44a"; 44b', 44b"; 54',54" jeweils eine Rastereinrichtung aufweisen, die es im Zusammenwirken der beiden Schenkel 14',14"; 24',24"; 44a', 44a"; 44b',44b"; 54',54" ermöglicht, den umgriffenen Endabschnitt 16; 26; 46a, 46b; 56 auf wählbaren diskreten räumlichen Positionen relativ zur **Schaftachse a** des länglichen Verbindungselements 13; 23; 43; 53 zu fixieren.

Die Rastereinrichtungen der Schenkel 14',14"; 24',24"; 44a', 44a"; 44b',44b"; 54',54" des Aufnahmeteils 14; 24; 44a, 44b; 54 auf den im implantierten Zustand der Gehörknöchelchenprothese 10; 20; 40; 50 am Außenumfang des Endabschnitts 16; 26; 46a, 46b; 56 anliegenden Innenflächen haben bei den in der Zeichnung dargestellten Ausführungsformen jeweils an gegenüberliegenden axialen Positionen der beiden Schenkel 14',14"; 24',24"; 44a', 44a"; 44b', 44b"; .54',54" parallel zur Schenkelachse äquidistant angeordnete Einkerbungen oder **Einbuchtungen 17; 27; 47a, 47b; 57** zur Aufnahme des Endabschnitts 16; 26; 46a, 46b; 56, welcher eine auf seinem Außenumfang azimutal verlaufende **Ringnut 18; 28; 48a, 48b; 58** aufweist, die geometrisch so gestaltet ist, dass sie als Gegenstück zu einer der Einkerbungen oder Einbuchtungen 17; 27; 47a, 47b; 57 der Rastereinrichtungen der beiden Schenkel 14',14"; 24',24"; 44a', 44a"; 44b',44b"; 54',54" des klammerartigen des Aufnahmeteils 14; 24; 44a, 44b; 54 passt.

Das Einschiebeteil 15; 25; 45a, 45b; 55 mit dem Aufnahmeteil 14; 24; 44a, 44b; 54 ist bei den gezeigten Ausführungsformen mittels der eigenen Federwirkung im Zusammenwirken der beiden Schenkel 14',14"; 24',24"; 44a', 44a"; 44b',44b"; 54',54" des Aufnahmeteils 14; 24; 44a, 44b; 54 passiv verklemmbar.

Alternativ oder zusätzlich kann das Einschiebeteil 15; 25; 45a, 45b; 55 mittels einer äußeren Einwirkung auf die beiden Schenkel 14',14"; 24',24"; 44a', 44a"; 44b',44b"; 54',54" aktiv mit dem Aufnahmeteil 14; 24; 44a, 44b; 54 verklemmt werden, etwa durch Krafteinwirkung von außen, insbesondere mittels Einwirkung einer Crimpzange. Möglich ist auch eine aktive Verklemmung durch Wärmeeintrag auf die Gehörknöchelchenprothese 10; 20; 40; 50 von außen, insbesondere mittels Erwärmung auf Körpertemperatur. Ganz besonders vorteilhaft ist es hierfür, wenn das Aufnahmeteil 14; 24; 44a, 44b; 54 und/oder das Einschiebeteil 15; 25; 45a, 45b; 55 ganz oder teilweise aus einem Material mit Formgedächtnis (=memory effect) und/oder superelastischen Eigenschaften, insbesondere aus Nitinol hergestellt sind.

Bei den Ausführungsformen der Figuren 1, 2 und 5 sind die beiden Schenkel 14',14"; 24',24"; 54',54" des Aufnahmeteils 14; 24; 54 im Wesentlichen parallel zur Schaftachse a des länglichen Verbindungselements 13; 23; 53 angeordnet.

In der Ausführungsform nach Figur 4 weist das Verbindungselement 43 mehrere Aufnahmeteile 44a, 44b mit jeweils im Wesentlichen parallelen Schenkeln 44a',44a"; 44b',44b" auf, welche jeweils einen Endabschnitt 46a, 46b eines zugehörigen Einschiebeteils 45a, 45b klammerartig umgreifen. Darüber hinaus ist bei dieser Ausführungsform in das Verbindungselement 43 ein **Kugelgelenk 49b** integriert, um eine gewisse postoperative Flexibilität der Gehörknöchelchenprothese 40 zwischen ihren Verbindungsstellen zu erreichen. Genauer gesagt ist das Einschiebeteil 45b an seinem dem Endabschnitt 46b abgewandten Ende über das Kugelgelenk 49b verschwenkbar mit dem zweiten Befestigungselement 42 verbunden. Über ein weiteres **Kugelgelenk** 49a ist das Aufnahmeteil 44a an seinem den beiden Schenkeln 44a',44a" abgewandten Ende verschwenkbar mit dem ersten Befestigungselement 41 verbunden, was die Beweglichkeit der Prothese noch weiter erhöht.

Bei der in Figur 1 gezeigten Ausführungsform ist das erste Befestigungselement 11 einer Kopfplatte zur Anlage am Trommelfell ausgebildet. Das zweite Befestigungselement 12 an dem der Kopfplatte entgegen gesetzten Ende ist in diesem Ausführungsbeispiel stempelförmig zur Auflage auf der Steigbügel-Fußplatte gestaltet. Letzteres trifft auch für die in Figur 4 dargestellte vierte Ausführungsform einer erfindungsgemäßen Gehörknöchelchenprothese 40 zu, wo das erste Befestigungselement 41 ebenfalls als Trommelfell-Kopfplatte ausgebildet ist.

Bei der Ausführungsform nach Figur 2 hingegen ist in das erste Befestigungselement 21 in Form einer Klammer ausgebildet, die beispielsweise auf den Ambossfortsatz oder auch auf ein anderes Glied der Gehörknöchelchenkette aufgeclipst werden kann. Das zweite Befestigungselement 22 ist als Kolben zur direkten Ankopplung der Gehörknöchelchenprothese 20 an das Innenohr gestaltet.

Bei der Ausführungsform nach Figur 5 ist das erste Befestigungselement 51 wiederum eine Trommelfell-Kopfplatte, während das zweite Befestigungselement 52 hier glockenartig gestaltet ist und vorzugsweise zur Befestigung der Gehörknöchelchenprothese 50 am Steigbügel dient. Das Aufnahmeteil 54 ist mit dem zweiten Befestigungselement 52 starr verbunden, während das Einschiebeteil 55 mit seinem Endabschnitt 56 Teil des ersten Befestigungselements 51 ist.

Die Massenverteilung der einzelnen Teile der erfindungsgemäßen Gehörknöchelchenprothese 10; 20; 40; 50 kann in Abhängigkeit von einem gewünschten, vorgebbaren Frequenzgang der Schallleitung im Mittelohr derart berechnet sein, dass ein individuelles akustisches Tuning der Schallleitungs-Eigenschaften ermöglicht wird. Dies kann auch - bei in der Zeichnung nicht dargestellten Ausführungsformen der Erfindung - durch an die Gehörknöchelchenprothese anclipsbare "Trimm-Massen" erreicht werden.

## Patentansprüche

1. Passive Gehörknöchelchenprothese (10; 20; 40; 50), die mindestens ein Glied der menschlichen Gehörknöchelchenkette ersetzt oder überbrückt, wobei die Gehörknöchelchenprothese (10; 20; 40; 50) an einem Ende ein erstes Befestigungselement (11; 21; 41; 51) zur mechanischen Verbindung mit dem Trommelfell oder einem Glied der Gehörknöchelchenkette, insbesondere mit dem Ambossfortsatz oder mit dem Hammergriff, und an ihrem anderen Ende ein zweites Befestigungselement (12; 22; 42; 52) zur mechanischen Verbindung mit einem weiteren Glied oder Teilen eines Gliedes der Gehörknöchelchenkette oder direkt mit dem Innenohr sowie ein die beiden Befestigungselemente (11; 21; 41 bzw. 12; 22; 42; 52) Schall leitend miteinander verbindendes, längliches Verbindungselement (13; 23; 43; 53) umfasst, welches ein als Aufnahmeteil (14; 24; 44a, 44b; 54) längs der Schaftachse (a) des länglichen Verbindungselements (13; 23; 43; 53) ausgebildetes erstes Teilstück und ein als in eine Aufnahmeöffnung des Aufnahmeteils (14; 24; 44a, 44b; 54) einschiebbares, mit dem Aufnahmeteil (14; 24; 44a, 44b; 54) verklemmbares Einschiebeteil (15; 25; 45a, 45b; 55) ausgebildetes zweites Teilstück aufweist, wobei das erste Befestigungselement (11; 21; 41; 51) mit einem Ende und das zweite Befestigungselement (12; 22; 42; 52) mit dem axial entgegen gesetzten anderen Ende des Verbindungselements (13; 23; 43; 53) mechanisch fest verbunden ist,
**dadurch gekennzeichnet,**
**dass** das Aufnahmeteil (14; 24; 44a, 44b; 54) geometrisch so gestaltet ist, dass es einen Endabschnitt (16; 26; 46a, 46b; 56) des Einschiebeteils (15; 25; 45a, 45b; 55) mit zwei gegenüber liegenden, im Wesentlichen parallelen und im Wesentlichen parallel zur Schaftachse (a) des länglichen Verbindungselements (13; 23; 53) angeordneten Schenkeln (14',14"; 24',24"; 44a', 44a"; 44b',44b"; 54',54") klammerartig kraft- und/oder formschlüssig umgreift,
**dass** die beiden Schenkel (14',14"; 24',24"; 44a', 44a"; 44b',44b"; 54',54") jeweils eine Rastereinrichtung aufweisen, die im Zusammenwirken der beiden Schenkel (14',14"; 24',24"; 44a', 44a"; 44b',44b"; 54',54") ermöglicht, den umgriffenen Endabschnitt (16; 26; 46a, 46b; 56) auf wählbaren diskreten räumlichen Positionen relativ zur Schaftachse (a) des länglichen Verbindungselements (13; 23; 43; 53) zu fixieren,
**dass** die Rastereinrichtungen der Schenkel (14',14"; 24',24"; 44a', 44a"; 44b',44b"; 54',54") des Aufnahmeteils (14; 24; 44a, 44b; 54) auf den im implantierten Zustand der Gehörknöchelchenprothese (10; 20; 40; 50) am Außenumfang des Endabschnitts (16; 26; 46a, 46b; 56) anliegenden Innenflächen jeweils an gegenüber liegenden axialen Positionen der beiden Schenkel (14',14"; 24',24"; 44a', 44a"; 44b',44b"; 54',54") Einkerbungen oder Einbuchtungen (17; 27; 47a, 47b; 57) zur Aufnahme des Endabschnitts (16; 26; 46a, 46b; 56) aufweisen,
und **dass** die Einkerbungen oder Einbuchtungen (17; 27; 47a, 47b; 57) auf den Innenflächen der beiden Schenkel (14',14"; 24',24"; 44a', 44a"; 44b',44b"; 54',54") jeweils in axialer Richtung der Schenkel (14',14"; 24',24"; 44a', 44a"; 44b',44b"; 54',54") äquidistant angeordnet sind.

2. Gehörknöchelchenprothese nach Anspruch 1, **dadurch gekennzeichnet, dass** das Verbindungselement (43) mindestens ein Gelenk, insbesondere ein Kugelgelenk (49a, 49b) aufweist.

3. Gehörknöchelchenprothese nach Anspruch 2, **dadurch gekennzeichnet, dass** das Aufnahmeteil (44a) an seinem den beiden Schenkeln (44a',44a") abgewandten Ende über ein Kugelgelenk (49a) verschwenkbar mit dem ersten Befestigungselement (41) verbunden ist.

4. Gehörknöchelchenprothese nach einem der Ansprüche 2 oder 3, **dadurch gekennzeichnet, dass** das Einschiebeteil (45b) an seinem dem Endabschnitt (46b) abgewandten Ende über ein Kugelgelenk (49b) verschwenkbar mit dem zweiten Befestigungselement (42) verbunden ist.

5. Gehörknöchelchenprothese nach einem der vorhergehenden Ansprüche, **dadurch gekennzeichnet, dass** das Verbindungselement (43) mehrere Aufnahmeteile (44a, 44b) mit jeweils im Wesentlichen parallelen Schenkeln (44a',44a"; 44b',44b") aufweist, welche jeweils einen Endabschnitt (46a, 46b) eines Einschiebeteils (45a, 45b) klammerartig umgreifen.

6. Gehörknöchelchenprothese nach einem der vorhergehenden Ansprüche, **dadurch gekennzeichnet, dass** der Endabschnitt (16; 26; 46a, 46b; 56) des Einschiebeteils (15; 25; 45a, 45b; 55) eine auf seinem Außenumfang azimutal verlaufende Ringnut (18; 28; 48a, 48b; 58) aufweist, die geometrisch so gestaltet ist, dass sie als Gegenstück zu einer der Einkerbungen oder Einbuchtungen (17; 27; 47a, 47b; 57) der Rastereinrichtungen der beiden Schenkel (14',14"; 24',24"; 44a', 44a"; 44b',44b"; 54',54") des klammerartigen Aufnahmeteils (14; 24; 44a, 44b; 54) passt.

7. Gehörknöchelchenprothese nach einem der Ansprüche 1 bis 6, **dadurch gekennzeichnet, dass** das Einschiebeteil (15; 25; 45a, 45b; 55) mit dem Aufnahmeteil (14; 24; 44a, 44b; 54) passiv verklemmbar ist mittels der eigenen Federwirkung im Zusammenwirken der beiden Schenkel (14',14"; 24',24"; 44a', 44a"; 44b',44b"; 54',54") des Aufnahmeteils (14; 24; 44a, 44b; 54).

8. Gehörknöchelchenprothese nach einem der Ansprüche 1 bis 6, **dadurch gekennzeichnet, dass** das Einschiebeteil (15; 25; 45a, 45b; 55) mit dem Aufnahmeteil (14; 24; 44a, 44b; 54) aktiv verklemmbar ist mittels einer äußeren Einwirkung auf die beiden Schenkel (14',14"; 24',24"; 44a', 44a"; 44b',44b"; 54',54") des Aufnahmeteils (14; 24; 44a, 44b; 54).

9. Gehörknöchelchenprothese nach Anspruch 8, **dadurch gekennzeichnet, dass** das Einschiebeteil (15; 25; 45a, 45b; 55) durch Krafteinwirkung von außen, insbesondere mittels Einwirkung einer Crimpzange, mit dem Aufnahmeteil (14; 24; 44a, 44b; 54) aktiv verklemmbar ist.

10. Gehörknöchelchenprothese nach Anspruch 8, **dadurch gekennzeichnet, dass** das Einschiebeteil (15; 25; 45a, 45b; 55) durch Wärmeeintrag auf die Gehörknöchelchenprothese (10; 20; 40; 50) von außen, insbesondere mittels Erwärmung der Gehörknöchelchenprothese (10; 20; 40; 50) auf Temperaturen bis zu 90°C, vorzugsweise auf Körpertemperatur, mit dem Aufnahmeteil (14; 24; 44a, 44b; 54) aktiv verklemmbar ist.

11. Gehörknöchelchenprothese nach Anspruch 10, **dadurch gekennzeichnet, dass** das Aufnahmeteil (14; 24; 44a, 44b; 54) und/oder das Einschiebeteil (15; 25; 45a, 45b; 55) ganz oder teilweise aus einem Material mit Formgedächtnis (=memory effect) und/oder mit superelastischen Eigenschaften, insbesondere aus Nitinol hergestellt sind.

## Claims

1. Passive ossicular prosthesis (10; 20; 40; 50) which replaces or bridges at least one component of the human ossicular chain, the ossicular prosthesis (10; 20; 40; 50) including at one end a first fastening element (11; 21; 41; 51) for mechanical connection to the tympanic membrane or to a component of the ossicular chain, in particular to the limb of the incus or to the manubrium of the malleus, and at its other end a second fastening element (12; 22; 42; 52) for mechanical connection to a further component or parts of a component of the ossicular chain, or directly to the inner ear, and including an elongated connecting element (13; 23; 43; 53) linking the two fastening elements (11; 21; 41; and 12; 22; 42; 52) in a sound-conducting manner, which connecting element (13; 23; 43; 53) has a first partial section configured as a receiving part (14; 24; 44a, 44b; 54) extending along the shaft axis (a) of the elongated connecting element (13; 23; 43; 53), and a second partial section configured as an insertable part (15; 25; 45a, 45b; 55) which can be inserted in a receiving opening of the receiving part (14; 24; 44a, 44b; 54) and can be locked to the receiving part (14; 24; 44a, 44b; 54), the first fastening element (11; 21; 41; 51) being mechanically rigidly connected to one end, and the second fastening element (12; 22; 42; 52) being mechanically rigidly connected to the axially opposite other end, of the connecting element (13; 23; 43; 53),
**characterised in that**
the receiving part (14; 24; 44a, 44b; 54) is configured geometrically in such a way that it encompasses with two opposed legs (14', 14"; 24', 24"; 44a', 44a"; 44b', 44b"; 54', 54"), with a nonpositive and/or positive fit in the manner of a clamp, an end section (16; 26; 46a, 46b; 56) of the insertable part (15; 25; 45a, 45b; 55), which legs (14', 14"; 24', 24"; 44a', 44a"; 44b', 44b"; 54', 54") are arranged substantially parallel to one another and substantially parallel to the shaft axis (a) of the elongated connecting element (13; 23; 53),
**in that** the two legs (14', 14"; 24', 24"; 44a', 44a"; 44b', 44b"; 54', 54") each have a latching arrangement which, in cooperation with the two legs (14', 14"; 24', 24"; 44a', 44a"; 44b', 44b"; 54', 54"), enables the encompassed end section (16; 26; 46a, 46b; 56) to be fixed in selectable discrete spatial positions relative to the shaft axis (a) of the elongated connecting element (13; 23; 43; 53),
**in that** the latching arrangements of the legs (14', 14"; 24', 24"; 44a', 44a"; 44b', 44b"; 54', 54") of the receiving part (14; 24; 44a, 44b; 54) have, on their inner faces which rest against the outer circumference of the end section (16; 26; 46a, 46b; 56) in the implanted state of the ossicular prosthesis (10; 20; 40; 50) at respective opposite axial positions of the two legs (14', 14"; 24', 24"; 44a', 44a"; 44b', 44b"; 54', 54"), notches or indentations (17; 27; 47a, 47b; 57) for receiving the end section (16; 26; 46a, 46b; 56),
and **in that** the notches or indentations (17; 27; 47a, 47b; 57) are each arranged on the inner faces of the two legs (14', 14"; 24', 24"; 44a', 44a"; 44b', 44b"; 54', 54") equidistantly in the axial direction of the legs (14', 14"; 24', 24"; 44a', 44a"; 44b', 44b"; 54', 54").

2. Ossicular prosthesis according to Claim 1, **characterised in that** the connecting element (43) has at least one joint, in particular a ball joint (49a, 49b).

3. Ossicular prosthesis according to Claim 2, **characterised in that** the receiving part (44a), at its end oriented away from the two legs (44a', 44a"), is connected swivellably via a ball joint (49a) to the first fastening element (41).

4. Ossicular prosthesis according to either of Claims 2 and 3, **characterised in that** the insertable part (45b), at its end oriented away from the end section (46b), is connected swivellably via a ball joint (49b) to the second fastening element (42).

5. Ossicular prosthesis according to any one of the preceding claims, **characterised in that** the connecting element (43) has a plurality of receiving parts (44a, 44b) each having respective substantially parallel arms (44a', 44a"; 44b', 44b"), each of which encompasses a respective end section (46a, 46b) of an insertable part (45a, 45b) in the manner of a clamp.

6. Ossicular prosthesis according to any one of the preceding claims, **characterised in that** the end section (16; 26; 46a, 46b; 56) of the insertable part (15; 25; 45a, 45b; 55) has on its outer circumference an azimuthally disposed annular groove (18; 28; 48a, 48b; 58) which is configured geometrically in such a way that fits as a counterpart one of the notches or indentations (17; 27; 47a, 47b; 57) of the latching arrangements of the two legs (14', 14"; 24', 24"; 44a', 44a"; 44b', 44b"; 54', 54") of the clamp-like receiving part (14; 24; 44a, 44b; 54).

7. Ossicular prosthesis according to any one of Claims 1 to 6, **characterised in that** the insertable part (15; 25; 45a, 45b; 55) can be clamped passively to the receiving part (14; 24; 44a, 44b; 54) by means of the inherent spring effect in the interaction of the two legs (14', 14"; 24', 24"; 44a', 44a"; 44b', 44b"; 54', 54") of the receiving part (14; 24; 44a, 44b; 54).

8. Ossicular prosthesis according to any one of Claims 1 to 6, **characterised in that** the insertable part (15; 25; 45a, 45b; 55) can be clamped actively to the receiving part (14; 24; 44a, 44b 54) by means of an external effect acting on the two legs (14', 14"; 24', 24"; 44a', 44a"; 44b', 44b"; 54', 54") of the receiving part (14; 24; 44a, 44b; 54).

9. Ossicular prosthesis according to Claim 8, **characterised in that** the insertable part (15; 25; 45a, 45b; 55) can be clamped actively to the receiving part (14; 24; 44a, 44b; 54) through the effect of an external force, in particular through the action of a crimping tool.

10. Ossicular prosthesis according to Claim 8, **characterised in that** the insertable part (15; 25; 45a, 45b; 55) can be actively clamped to the receiving part (14; 24; 44a, 44b; 54) through the application of heat to the ossicular prosthesis (10; 20; 40; 50) from outside, in particular through heating of the ossicular prosthesis (10; 20; 40; 50) to temperatures up to 90°C, preferably to body temperature.

11. Ossicular prosthesis according to Claim 10, **characterised in that** the receiving part (14; 24; 44a, 44b; 54) and/or the insertable part (15; 25; 45a, 45b; 55) is/are produced wholly or partially from a material having memory effect and/or having superelastic properties, in particular from nitinol.

## Revendications

1. Prothèse passive des osselets auditifs (10 ; 20 ; 40 ; 50), qui remplace ou ponte au moins un composant de la chaîne humaine des osselets auditifs, la prothèse des osselets auditifs (10 ; 20 ; 40 ; 50) comprenant à une extrémité un premier élément de fixation (11 ; 21 ; 41 ; 51) pour le raccordement mécanique au tympan ou à un composant de la chaîne des osselets auditifs, en particulier à l'enclume ou au manche du marteau et, à son autre extrémité, un deuxième élément de fixation (12 ; 22 ; 42 ; 52) pour le raccordement mécanique à un autre composant ou des parties d'un composant de la chaîne des osselets auditifs ou directement à l'oreille interne, ainsi qu'un élément de raccordement (13 ; 23 ; 43 ; 53) oblong qui raccorde entre eux les deux éléments de fixation (11 ; 21 ; 41 ou respectivement 12 ; 22 ; 42 ; 52) de façon acoustiquement conductrice et qui présente une première pièce partielle constituée en tant que partie de réception (14 ; 24 ; 44a, 44b ; 54) le long de l'axe de tige (a) de l'élément de raccordement (13 ; 23 ; 43 ; 53) oblong et une deuxième pièces partielle constituée en tant que partie coulissante (15 ; 25 ; 45a, 45b ; 55) pouvant être coincée avec la partie de réception (14 ; 24 ; 44a, 44b ; 54) et pouvant coulisser dans une ouverture de réception de la partie de réception (14 ; 24 ; 44a, 44b ; 54), le premier élément de fixation (11 ; 21 ; 41 ; 51) étant raccordé de façon mécaniquement fixe à une extrémité, et le deuxième élément de fixation (12 ; 22 ; 42 ; 52) étant raccordé de façon mécaniquement fixe à l'autre extrémité, opposée axialement, de l'élément de raccordement (13 ; 23 ; 43 ; 53),
**caractérisée en ce que**
la partie de réception (14 ; 24 ; 44a, 44b ; 54) est constituée géométriquement de telle sorte qu'elle entoure à la façon d'une agrafe, par liaison de force et/ou par liaison de forme, un tronçon extrême (16 ; 26 ; 46a, 46b ; 56) de la partie coulissante (15 ; 25 ; 45a, 45b ; 55) avec deux branches (14', 14" ; 24', 24" ; 44a', 44a" ; 44b', 44b" ; 54', 54") opposées, essentiellement parallèles et essentiellement parallèlement à l'axe de tige (a) de l'élément de raccordement (13 ; 23 ; 53) oblong,
**en ce que** les deux branches (14', 14" ; 24', 24" ; 44a', 44a" ; 44b', 44b" ; 54', 54") présentent respectivement un dispositif d'encliquetage qui, en coopération avec les deux branches (14', 14" ; 24', 24" ; 44a', 44a" ; 44b', 44b" ; 54', 54"), permet de fixer le tronçon extrême (16 ; 26; 46a, 46b ; 56) entouré dans des positions spatiales discrètes sélectionnables par rapport à l'axe de tige (a) de l'élément de raccordement (13 ; 23 ; 43 ; 53) oblong,
**en ce que** les dispositifs d'encliquetage des branches (14', 14" ; 24', 24" ; 44a', 44a" ; 44b', 44b" ; 54', 54") de la partie de réception (14 ; 24 ; 44a, 44b ; 54) présentent, sur les surfaces intérieures adjacentes, dans l'état implanté de la prothèse des osselets auditifs (10 ; 20 ; 40 ; 50), contre la circonférence extérieure du tronçon extrême (16 ; 26 ; 46a, 46b ; 56), respectivement des entailles ou des échancrures (17 ; 27 ; 47a, 47b ; 57) dans des positions axiales opposées des deux branches (14', 14" ; 24', 24" ; 44a', 44a" ; 44b', 44b" ; 54', 54") pour la réception du tronçon extrême (16 ; 26 ; 46a, 46b ; 56),
et **en ce que** les entailles ou échancrures (17 ; 27 ; 47a, 47b ; 57) sont disposées de façon équidistante sur les surfaces intérieures des deux branches (14', 14" ; 24', 24" ; 44a', 44a" ; 44b', 44b" ; 54', 54") respectivement dans la direction axiale des branches (14', 14" ; 24', 24" ; 44a', 44a" ; 44b', 44b" ; 54', 54").

2. Prothèse des osselets auditifs selon la revendication 1, **caractérisée en ce que** l'élément de raccordement (43) présente au moins une articulation, en particulier une articulation à rotule (49a, 49b).

3. Prothèse des osselets auditifs selon la revendication 2, **caractérisée en ce que** la partie de réception (44a) est, à son extrémité éloignée des deux branches (44a', 44a"), raccordée de façon pivotante au premier élément de fixation (41) par le biais d'une articulation à rotule (49a).

4. Prothèse des osselets auditifs selon une des revendications 2 ou 3, **caractérisée en ce que** la partie coulissante (45b) est, à son extrémité éloignée du tronçon extrême (46b), raccordée de façon pivotante au deuxième élément de fixation (42) par le biais d'une articulation à rotule (49b).

5. Prothèse des osselets auditifs selon une des revendications précédentes, **caractérisée en ce que** l'élément de raccordement (43) présente plusieurs parties de réception (44a, 44b) avec respectivement des branches (44a', 44a" ; 44b', 44b") essentiellement parallèles qui entourent à la façon d'une agrafe respectivement un tronçon extrême (46a, 46b) d'une partie coulissante (45a, 45b).

6. Prothèse des osselets auditifs selon une des revendications précédentes, **caractérisée en ce que** le tronçon extrême (16 ; 26 ; 46a, 46b ; 56) de la partie coulissante (15 ; 25 ; 45a, 45b ; 55) présente sur sa circonférence extérieure une rainure annulaire (18 ; 28 ; 48a, 48b ; 58) s'étendant de façon azimutale qui est géométriquement constituée de telle sorte qu'elle s'adapte, en tant que pendant, à une des entailles ou des échancrures (17 ; 27 ; 47a, 47b ; 57) des dispositifs d'encliquetage des deux branches (14', 14" ; 24', 24" ; 44a', 44a" ; 44b', 44b" ; 54', 54") de la partie de réception (14 ; 24 ; 44a, 44b ; 54) de type agrafe.

7. Prothèse des osselets auditifs selon une des revendications 1 à 6, **caractérisée en ce que** la partie coulissante (15 ; 25 ; 45a, 45b ; 55) peut être coincée passivement avec la partie de réception (14 ; 24 ; 44a, 44b ; 54) au moyen de la propre action de ressort lors de la coopération des deux branches (14', 14" ; 24', 24" ; 44a', 44a" ; 44b', 44b" ; 54', 54") de la partie de réception (14 ; 24 ; 44a, 44b ; 54).

8. Prothèse des osselets auditifs selon une des revendications 1 à 6, **caractérisée en ce que** la partie coulissante (15 ; 25 ; 45a, 45b ; 55) peut être coincée activement avec la partie de réception (14 ; 24 ; 44a, 44b ; 54) au moyen d'une action extérieure agissant sur les deux branches (14', 14" ; 24', 24" ; 44a', 44a" ; 44b', 44b" ; 54', 54") de la partie de réception (14 ; 24 ; 44a, 44b ; 54).

9. Prothèse des osselets auditifs selon la revendication 8, **caractérisée en ce que** la partie coulissante (15 ; 25 ; 45a, 45b ; 55) peut, par l'action d'une force exercée à partir de l'extérieur, en particulier au moyen de l'action d'une pince à sertir, être coincée activement avec la partie de réception (14 ; 24 ; 44a, 44b ; 54).

10. Prothèse des osselets auditifs selon la revendication 8, **caractérisée en ce que** la partie coulissante (15 ; 25 ; 45a, 45b ; 55) est coincée activement avec la partie de réception (14 ; 24 ; 44a, 44b ; 54) par apport de chaleur à la prothèse des osselets auditifs (10 ; 20 ; 40 ; 50) à partir de l'extérieur, en particulier en chauffant la prothèse des osselets auditifs (10 ; 20 ; 40 ; 50) à des températures jusqu'à 90°C, de préférence jusqu'à la température corporelle.

11. Prothèse des osselets auditifs selon la revendication 10, **caractérisée en ce que** la partie de réception (14 ; 24 ; 44a, 44b ; 54) et/ou la partie coulissante (15 ; 25 ; 45a, 45b ; 55) sont réalisées, totalement ou partiellement, en un matériau à mémoire de forme (= memory effect) et/ou présentant des propriétés super élastiques, en particulier en nitinol.
